Europäisches Patentamt

European Patent Office

Office européen des brevets

(11) Publication number: **0 215 588**
**A2**

# (12) EUROPEAN PATENT APPLICATION

(21) Application number: 86306557.9

(22) Date of filing: 22.08.86

(51) Int. Cl.⁴: **C 07 C 29/90**
C 07 C 31/12,
C 07 C 29/132, C 10 L 1/02

(30) Priority: 16.09.85 US 776339  19.09.85 US 777795
06.03.86 US 836799  06.03.86 US 836798

(43) Date of publication of application:
25.03.87 Bulletin 87/13

(84) Designated Contracting States: BE DE FR GB NL

(71) Applicant: TEXACO DEVELOPMENT CORPORATION
2000 Westchester Avenue
White Plains New York 10650 (US)

(72) Inventor: Sanderson, John Ronald
5306 Rambling Range
Austin Texas 78759 (US)

Labkin, John Michael
12414 Dorsett Road
Austin Texas 78759 (US)

Marquis, Edward Thomas
9004 Collinfield Drive
Austin Texas 78758 (US)

Keating, Kenneth Patrick
204 Oakwood Drive
Georgetown Texas 78626 (US)

(74) Representative: Burnside, Michael et al
Michael Burnside & Partners 2 Serjeants' Inn Fleet Street
London EC4Y 1HL (GB)

(54) Removal of residual peroxide from tertiary butyl alcohol.

(57) A motor fuel grade tertiary butyl alcohol feedstock obtained by the reaction of propylene with tertiary butyl hydroperoxide which is contaminated with a residual amout of tertiary butyl hydroperoxide can be effectively catalytically treated with a supported, nickel, palladium and/or platinum catalyst in the presence of added hydrogen under mild catalytic reduction conditions including a temperature of about 50° to 150°C and a pressure of up to about 70 kg/cm² (1000 psig) in order to s bstantially selectively convert the hydroperoxide contaminants to tertiary butyl alcohol and to thereby provide a treated product substantially free from contaminating quantities of tertiary butyl hydroperoxide.

EP 0 215 588 A2

**Description**

REMOVAL OF RESIDUAL PEROXIDE FROM TERTIARY BUTYL ALCOHOL.

This invention relates to the catalytic purification of tertiary butyl alcohol prepared by the reaction of propylene with tertiary butyl hydroperoxide. More particularly, this invention relates to a method for the removal of residual contaminating quantities of tertiary butyl hydroperoxide and ditertiary butyl peroxide from a tertiary butyl alcohol feedstock prepared by the reaction of propylene with tertiary butyl hydroperoxide useful as an octane-enhancing component for motor fuels wherein the peroxide-contaminated feedstock is brought into contact in the presence of hydrogen under mild catalytic reduction conditions with a catalyst supported on a base preferably consisting essentially of about 50 to 100 wt. % of alumina and 0 to about 50 wt.% of silica in order to substantially selectively reduce the tertiary butyl hydroperoxide and the ditertiary butyl peroxide to tertiary butyl alcohol.

A process for the manufacture of substituted epoxides from alpha olefins such as propylene is disclosed in Kollar U.S. Patent No. 3,351,653 which teaches that an organic epoxide compound can be made by reacting an olefinically unsaturated compound with an organic hydroperoxide in the presence of molybdenum, tungsten, titanium, columbium, tantalum, rhenium, selenium, chromium, zirconium, tellurium or uranium catalyst. When the olefin is propylene and the hydroperoxide is tertiary butyl hydroperoxide, propylene oxide and tertiary butyl alcohol are coproducts. U. S. Patent No. 3,350,422 teaches a similar process using a soluble vanadium catalyst. Molybdenum is the preferred catalyst. A substantial excess of olefin relative to the hydroperoxide is taught as the normal procedure for the reaction. See also U. S. Patent No. 3,526,645 which teaches the slow addition of organic hydroperoxide to an excess of olefin as preferred.

Stein, et al. in U. S. Patent No. 3,849,451 have improved upon the Kollar process of U. S. Patent Nos. 3,350,422 and 3,351,635 by requiring a close control of the reaction temperature, between 90-200°C and autogeneous pressures, among other parameters. Stein et al. also suggests the use of several reaction vessels with a somewhat higher temperature in the last vessel to ensure more complete reaction. The primary benefits are stated to be improved yields and reduced side reactions.

It is known that isobutane can be oxidized with molecular oxygen to form a corresponding tertiary butyl hydroperoxide and that the oxidation reaction can be promoted, for example with an oxidation catalyst (see Johnston U. S. Patent No. 3,825,605 and Worrell U. S. Patent No. 4,296,263.

Thus, tertiary butyl alcohol can be prepared either by the direct thermal or catalytic reduction of tertiary butyl hydroperoxide to tertiary butyl alcohol or by the catalytic reaction of propylene with tertiary butyl hydroperoxide to provide propylene oxide and tertiary butyl alcohol.

It is also known that tertiary butyl alcohol can be used as an octane-enhancing component when added to a motor fuel, such as gasoline. Thus, it has heretofore been proposed, as shown, for example, by Grane U. S. Patent No. 3,474,151 to thermally decompose tertiary butyl hydroperoxide and ditertiary butyl peroxide to form tertiary butyl alcohol. The thermal decomposition must be conducted with care, as pointed out by Grane, in that tertiary butyl alcohol will start to dehydrate at a temperature of about 450°F and in that the dehydration becomes rapid at temperatures above about 475°F. Moreover, the product from the thermal decomposition normally contains a minor amount of tertiary butyl hydroperoxide and ditertiary butyl peroxide which have an adverse effect upon the quality of motor fuels and must be substantially completely removed if the tertiary butyl alcohol is to be fully effective. Grane proposes to accomplish this thermally by heating tertiary butyl alcohol containing small quantities of such peroxides at a temperature of 375-475°F for a period of 1 to 10 minutes.

This concept was expanded upon by Grane et al. in U. S. Patent Nos. 4,294,999 and 4,296,262 to provide integrated processes wherein, starting with isobutane, motor-fuel grade tertiary butyl alcohol was prepared by the oxidation of isobutane (e.g., in the presence of a solubilized molybdenum catalyst) to produce a mixture of tertiary butyl alcohol and tertiary butyl hydroperoxide from which a fraction rich in tertiary butyl hydroperoxide could be recovered by distillation. This stream, after being debutanized was subjected to thermal decomposition under pressure at a temperature of less than 300°F for several hours to significantly reduce the concentration of the tertiary butyl hydroperoxide. However, the product of this thermal decomposition step still contained residual tertiary butyl hydroperoxide, most of which was thereafter removed by a final thermal treatment of the contaminated tertiary butyl hydroperoxide in the manner taught by Grane U. S. Patent No. 3,474,151.

Thus, the removal of trace quantities of tertiary butyl hydroperoxide from motor grade tertiary butyl alcohol has received appreciable attention. However, little appears to have been published concerning the removal of trace quantities of ditertiary butyl peroxide, the more refractory of the two peroxides. This may be explainable both because ditertiary butyl peroxide is not always present in trace quantities in motor grade tertiary butyl alcohol (its presence or absence being a function of the reaction conditions used in oxidizing the isobutane starting material) and because, when present, it is present in significantly lower amounts. For example, after decomposition of the major amount of tertiary butyl hydroperoxide formed by the oxidation of isobutane, the tertiary butyl hydroperoxide residual content will normally be about 0.1 to about 1 wt.%, based on the tertiary butyl alcohol, while the residual ditertiary butyl peroxide content, if any, will only be about 0.1 to 0.5 wt.%.

It has also been proposed to remove the residual hydroperoxide contaminants from tertiary butyl alcohol through the use of a heterogeneous cobalt oxide catalyst containing a copper oxide promoter as shown, for example, by Coile U. S. Patent No. 4,059,598. Allison et al. in U. S. Patent No. 3,505,360 have more generically taught that alkenyl hydroperoxides can be decomposed catalytically through the use of a catalyst based on a metal or compound of a metal of group IV-A, V-A or VI-A.

In their U. S. Patent No. 2,491,926, directed to the catalytic hydrogenation of $\alpha,\alpha$-dialkylarylmethyl hydroperoxides to prepare $\alpha,\alpha$-dialkylarylmethyl alcohols, Lorand et al. teach the use of supported and unsupported noble metals such as platinum, palladium and rhodium and heavy metal catalysts such as nickel and cobalt as catalysts, but do not discuss in detail the nature of the support to be used. They mention in passing that when diisopropyl benzene is oxidized, $\alpha,\alpha,\alpha',\alpha'$-tetramethyl-p-xylylene dihydroperoxide may be obtained. Balandin et al. have published papers discussing the use of nickel catalysts in the hydrogenation of peroxides and hydroperoxides. See, for example, "Kinetics of the Catalytic Reduction of Peroxides and hydroperoxides -Communication 2. Hydrogenation of Benzoyl Peroxide, tert-Butyl Peroxybenzoate, and 2-Cyclohexen-1-yl-hydroperoxide", A. A. Balandin et al. (Bulletin of the Academy of Science of the USSR, Division of Chemical Science (English Translation, 1958) and "Kinetics of the Catalytic Reduction of Peroxides and Hydroperoxides - III. Hydrogenation of 3-Methyl-1-butyne, 3-Hydroperoxide and p-Nitrobenzoyl Peroxide" (Bulletin of the Academy of Science of the USSR, Division of Chemical Science, English Translation, 1959).

Other prior art patents relating to the production of hydroperoxides, but not with the problem of residual tertiary hydroperoxide contamination and tertiary butyl alcohol include patents such as Rust U. S. Patent No. 2,383,919; Harvey U. S. Patent No. 3,449,217; Poenisch et al. U. S. Patent No. 3,778,382 and Williams et al. U. S. Patent No. 3,816,548.

In West German DE 3248465-A a two-step process is disclosed wherein isobutane is oxidized noncatalytically with air to a conversion of about 48-90% to form the corresponding hydroperoxide, which is then catalytically decomposed under hydrogenation conditions in the presence of a supported catalyst such as palladium, platinum, copper, rhenium, ruthenium or nickel to form tertiary butyl alcohol. The decomposition product obtained using 1.3% palladium on lithium spinel as a catalyst contained significant quantities of acetone, water and methanol.

Mabuchi et al. U. S. Patent No. 4,112,004 discloses a process for preparing monohydric or polyhydric alcohols from organic peroxides in the presence of a nickel catalyst by continuously feeding a solution of the organic peroxide (e.g., butadiene peroxide) and a suspension of the nickel catalyst to a reactor in a controlled ratio and continuously withdrawing reaction mixture at a rate adequate to maintain a constant weight and composition of the reaction mixture in the reactor.

In U. S. Patent No. 4,123,616 to Mabuchi et al. a process is disclosed for hydrogenating an organic peroxide to the corresponding mono- or polyhydric alcohol in a suspension or fluidized bed process under hydrogen pressure in the presence of a nickel catalyst. Examples are given showing the conversion of butadiene peroxide to 1,4-butane diol and 1,2-butane diol and the conversion of tertiary butyl hydroperoxide to tertiary butyl alcohol.

Quin U. S. Patent No. 2,854,487 is directed to the use of a catalyst composed of palladium supported on activated aluminium for the reduction of organic hydroperoxides to the corresponding carbinols. It is pointed out that hydrogenation catalysts can also catalyze the decomposition of organic peroxides into decomposition products such as ketones and aldehydes. Quin found that the use of palladium supported on activated alumina avoided this and related difficulties, particularly when reducing isopropylbenzene hydroperoxide to phenyldimethyl carbinol.

The catalytic oxidation of methyl-substituted aromatic hydrocarbons with organic hydroperoxides in the presence of a salt of a metal having an atomic number of 23 to 29 is disclosed in Rosenthal U. S. Patent No. 3,914,295. British Patent No. 1,018,661 discloses a process for the selective hydrogenation of organic hydroperoxides such as olefin hydroperoxides in the presence of other organic compounds, such as olefins, with hydrogen using a supported platinum group metal catalyst modified by the inclusion of another metal such as lead, mercury, cadmium, thallium, etc.

## SUMMARY OF INVENTION

In accordance with the present invention, tertiary butyl alcohol (which can be prepared, for example, by the oxidation of isobutane with molecular oxygen) is reacted with propylene oxide in a process of the type disclosed in Kollar U. S. Patent No. 3,351,635 to provide an initial reaction product composed mostly of unreacted propylene, propylene oxide and tertiary butyl alcohol. However, residual quantities of tertiary butyl hydroperoxide and ditertiary butyl hydroperoxide may be present and will remain dissolved in the tertiary butyl alcohol recovered from the reaction mixture. Tertiary butyl hydroperoxide can also decompose thermally and/or catalytically to form acetone which, in turn, can decompose to form isopropyl alcohol. Tertiary butyl alcohol can be decomposed to form isobutane. Accordingly, the tertiary butyl alcohol, as initially recovered is not entirely satisfactory for use as an octane-enhancing component for motor fuels, such as gasoline. Thus, tertiary butyl alcohol will normally be considered unsatisfactory for motor fuel use if it contains more than about 3 wt.% of acetone, more than about 1 wt.% of isobutane and/or n-butane, more than about 0.3 wt.% of isopropyl alcohol and/or more than about 1 wt.% of methanol and/or methyl formate. Desirably, the tertiary butyl alcohol will contain about 1 wt.% or less of acetone, 0.5 wt.% or less of isobutane and/or n-butane, about 0.1 wt.% or less of isopropyl alcohol and about 0.5 wt.% or less of methanol and/or methyl formate.

It has been surprisingly discovered in accordance with the present invention that motor-fuel grade tertiary butyl alcohol which is contaminated with a residual amount of ditertiary butyl peroxide and tertiary butyl

3

hydroperoxide can be effectively catalytically treated with an appropriately supported catalyst.

According to the present invention therefore there is provided a method for enhancing the motor fuel quality of a tertiary butyl alcohol feedstock obtained by the reaction of propylene with tertiary butyl hydroperoxide to form propylene oxide and tertiary butyl alcohol contaminated with residual quantities of tertiary butyl hydroperoxide, and ditertiary butyl peroxide where present, characterised by contacting said feedstock with a supported catalyst in the presence of hydrogen under mild catalytic conditions including a temperature of about 50° to 150°C, a maximum pressure of 70 kg/cm$^2$ (1000 psig) correlated to substantially selectively reduce said hydroperoxides to tertiary butyl alcohol and recovering substantially hydroperoxide-free tertiary butyl alcohol from the reaction products; wherein the catalyst is selected from; a nickel catalyst composition containing 5 to 75 wt % of the active catalytic metal component, and wherein said support comprises 50 to 100 wt % of alumina, and correspondingly 50 to 0 wt % of silica, and the minimum pressure is about 10.5 kg/cm$^2$ (150 psig);
or
a platinum and/or palladium catalyst composition containing 0.01 wt % to 5 wt % of active catalytic metal component, wherein the minimum pressure is 21 kg/cm$^2$ (300 psig).

The results obtained with the process of the present invention are surprising and unexpected in several respects. Catalytic or thermal decomposition of tertiary butyl hydroperoxide and ditertiary butyl peroxide normally results in the formation of acetone as the favored decomposition product. Thus, the favorable effect on the quality of motor fuel grade tertiary butyl alcohol that is obtained by the substantially complete elimination of hydro peroxides will be largely counterbalanced if the decomposition product is principally acetone. Moreover, acetone can be further reduced catalytically to isopropyl alcohol while tertiary butyl alcohol can be catalytically reduced to isobutane.

Moreover, we have found that catalysts that are effective for the substantially complete decomposition of tertiary butyl hydroperoxide are normally only partially effective, at best, for the catalytic reduction of ditertiary butyl peroxide. We have also found that the "inert" supports that are normally suggested for use with heavy metal catalysts can, and do, exert an unexpected influence on the course of the reactions involved in the catalytic reduction of ditertiary butyl peroxide. We obtained reasonably satisfactory results only with the selected catalysts and were surprised to discover that silica could also be incorporated into the support up to a level of about 50 wt.% of the total weight of the support in some instances.

Thus, the provision of the process of the present invention wherein a motor-fuel grade tertiary butyl alcohol feedstock containing contaminating quantities of both ditertiary butyl hydroperoxide and tertiary butyl hydroperoxide, is catalytically treated for the reduction of the peroxides so that they are substantially completely removed without significantly adding to the level of contamination due to acetone, isopropyl alcohol and isobutane constitutes a distinct advantage over the prior art.

STARTING MATERIALS

The starting materials for the process of the present invention include a motor-fuel grade tertiary butyl alcohol feedstock obtained by the reaction of propylene with tertiary butyl hydroperoxide to form propylene oxide and tertiary butyl alcohol.

The motor-fuel grade tertiary butyl alcohol feedstock obtained by the reaction of propylene with tertiary butyl hydroperoxide to form propylene oxide and tertiary butyl alcohol will contain contaminating quantities of teritary butyl hydroperoxide, ditertiary butyl hydroperoxide, acetone, isopropyl alcohol and isobutane. The normal levels of contamination of such materials are such that the tertiary butyl alcohol will normally contain, prior to treatment, from about 0.1 to about 5 wt.% of tertiary butyl hydroperoxide, from about 0.1 to about 5 wt.% of acetone, from about 0.1 to about 1 wt.% of isopropyl alcohol and from about 0.1 to about 5 wt.% of isobutane. Minor quantities of other contaminants may also be present.

As indicated earlier, the reaction conditions used in the catalytic oxidation of isobutane will sometimes result in the formation of ditertiary butyl hydroperoxide. Thus, the feedstock to be used for the practice of the present invention maybe an impure motor grade tertiary butyl alcohol containing from about 0.1 to about 1 wt.% of tertiary butyl hydroperoxide and from about 0.1 to about 0.5 wt.% of ditertiary butyl peroxide.

The nickel catalysts to be used in the practice of the process of the present invention are catalysts wherein the active catalytic component consists essentially of nickel and wherein from about 5 to about 75 wt.% of the catalyst composition is nickel and the remainder consists essentially of a support composed of from about 50 to 100% of alumina and from about 50 to 0 wt.% of silica.

Catalytic Treatment of Tertiary Butyl Alcohol with an Mi catalyst

In accordance with the present invention, a feedstock, as above described, is brought into contact with a nickel catalyst of the present invention in the presence of added hydrogen under catalytic reduction conditions correlated to substantially selectively reduce both the tertiary butyl hydroperoxide and ditertiary butyl peroxide contaminants in the tertiary butyl alcohol feedstock to tertiary butyl alcohol with not more than a minor increase in the level of contamination of the acetone, isopropyl alcohol and isobutane contaminants also normally present in the tertiary butyl alcohol.

The reaction may be conducted batchwise in an autoclave using powdered catalyst or may be conducted on a continuous basis by passing the tertiary butyl alcohol and hydrogen through a reactor containing a bed of a supported nickel catalyst of the present invention under mild catalytic reduction conditions. Thus, the temperature to be used is preferably a temperature within the range of about 50° to 150°C and, more preferably, from about 70° to 130°C. The reaction should be conducted under pressure, in order to maintain the tertiary butyl alcohol in liquid phase, the pressure suitably being from about 3.5 kg/cm$^2$ to 70 kg/cm$^2$ (50 to 1000 psig) more preferably, from about 10.5 kg/cm$^2$ to 49 kg/cm$^2$ (150 to 700 psig When the reaction is conducted batchwise, contact time may suitably be from about 0.5 to 4 hours. When the reaction is conducted on a continuous basis, the tertiary butyl alcohol should be passed over the bed of catalyst at a liquid hourly space velocity of about 0.25 to 5.

The amount of hydrogen utilized in the process is preferably within the range of about 1.0 to 10 moles, per mole of tertiary butyl hydroperoxide and ditertiary butyl peroxide (combined) and, more preferably, from 1.1 to 1.5. This amount of hydrogen can normally be provided by pressuring the reactor to the desired reaction pressure with hydrogen.

More vigorous catalytic reduction conditions such as a temperature of 180° to 250°C and 140 kg/cm$^2$ to 700 kg/cm$^2$ (2000 to 10,000 psig) hydrogen should be avoided.

The reaction product, after being degassed, is suitable for use as an octane-enhancing component of motor fuel, such as gasoline.

Thus, for example, the effluent from the reactor may be passed through a phase separation zone in order to permit gaseous reaction components including hydrogen and isobutane to volatilize from the product to thereby provide the desired reaction product.

The specific correlation of conditions to be utilized with any specific nickel catalyst can be determined by one of ordinary skill in the art with comparative ease. Thus, for example, the tertiary butyl alcohol feedstock should be analyzed prior to catalytic treatment to determine the level of contamination by tertiary butyl hydroperoxide, ditertiary butyl peroxide, acetone, isopropyl alcohol and isobutane. If there is an insufficient reduction of the hydroperoxides such that a significant amount (e.g., more than about 0.1 wt.%) of tertiary butyl hydroperoxide and/or ditertiary butyl peroxide is still present, the reaction conditions are not sufficiently severe, and should be increased such as, for example, by increasing reaction temperature or contact time in order to obtain the desired reduction of the tertiary butyl hydroperoxide.

If, on the other hand, there is a significant increase in the level of contamination of acetone, isopropyl alcohol and/or isobutane, the reaction conditions are too severe for the particular catalyst and the reaction conditions should be ameliorated (e.g., by reducing contact time or temperature).

The palladium and/or platinum catalysts to be used in accordance with the present invention are catalysts wherein the active catalytic component consists essentially of palladium, platinum or a mixture of the two metals and which are supported on an inert carrier such as carbon, silica, alumina, calcium carbonate, barium carbonate, barium sulfate, graphite, keisgelgher or spinel.

The palladium and/or platinum will suitably constituted from about 0.01 to about 5 wt.% of the catalyst composition and, more preferably, from about 0.5 to about 5 wt.%. Other metals known to promote the effectiveness of palladium as a reductive hydrogenation catalyst may be present in minor amounts such as, for example, sodium, potassium, rubidium, copper, silver, etc. A preferred catalyst consists essentially of palladium supported on an inert carrier of the type described above in the indicated proportions.

Catalytic Treatment of Tertiary Butyl Alcohol with Pt and Pd catalysts

In accordance with a preferred embodiment of the present invention, a feedstock, as above described, is brought into contact with a palladium and/or platinum catalyst of the present invention in the presence of added hydrogen under catalytic reduction conditions correlated to substantially selectively reduce the tertiary butyl hydroperoxide contaminant in the tertiary butyl alcohol feedstock to tertiary butyl alcohol with not more than a minor increase in the level of contamination of the acetone, isopropyl alcohol and isobutane contaminants also normally present in the tertiary butyl alcohol.

The mild catalytic reduction reaction may be conducted batchwise in an autoclave using powdered catalyst or may be conducted on a continuous basis by passing the tertiary butyl alcohol feedstock and hydrogen through a reactor containing a bed of pilled palladium or platinum catalyst. The temperature to be used is preferably a temperature within the range of about 50° to 150°C and, more preferably, from about 70° to about 130°C. The reaction should be conducted under pressure, in order to maintain the tertiary butyl alcohol feedstock in liquid phase, the pressure suitably being from 21 kg/cm$^2$ to 700 kg/cm$^2$ (300 to 1000 psig) and, more preferably, from about 21 kg/cm$^2$ to 49 kg/cm$^2$ (300 to 700 psig). When the reaction is conducted batchwise, contact time may suitably be from 0.5 to 4 hours.

When the reaction is conducted on a continuous basis, the tertiary butyl alcohol should be passed over the bed of catalyst at a liquid hourly space velocity of 0.25 to 5.

The amount of hydrogen utilized in the process is preferably within the range of 1.0 to 10 moles, per mole of tertiary butyl hydroperoxide and, more preferably, from 1.1 to 1.5. This amount of hydrogen can normally be provided by pressuring the reactor to the desired reaction pressure with hydrogen.

More vigorous catalytic reduction conditions should be avoided (e.g., a temperature of 100°-250°C and 140 kg/cm$^2$ to 700 kg/cm$^2$ (2,000 to 10,000 psig) hydrogen.

The reaction product, after being degassed, is suitable for use as an octane-enhancing component of motor fuel, such as gasoline.

Thus, for example, the effluent from the reactor may be passed through a phase separation zone in order to permit gaseous reaction components including hydrogen and isobutane to volatilize from the product to

5

thereby provide the desired reaction product.

The specific correlation of conditions to be utilized with any specific palladium catalyst can be determined by one of ordinary skill in the art with comparative ease. Thus, for example, the tertiary butyl alcohol feedstock should be analyzed prior to catalytic treatment to determine the level of contamination by tertiary butyl hydroperoxide, acetone, isopropyl alcohol and isobutane. If there is an insufficient reduction of the tertiary butyl hydroperoxide such that a significant amount (e.g., more than about 0.1 wt.%) of tertiary butyl hydroperoxide is still present, the reaction conditions are not sufficiently severe, and should be increased such as, for example, by increasing reaction temperature or contact time in order to obtain the desired reduction of the tertiary butyl hydroperoxide.

If, on the other hand, there is a significant increase in the level of contamination of acetone, isopropyl alcohol and/or isobutane, the reaction conditions are too severe for the particular catalyst and the reaction conditions should be ameliorated (e.g., by reducing contact time or temperature).

The invention will now be described by way of illustration only, with reference to the following examples:

## WORKING EXAMPLES

### Example A

Propylene was epoxidized with tert-butyl hydroperoxide prepared by the oxidation of isobutane. The catalyst and procedure was similar to the procedure given in Serial No. 06/797,079 filed November 15, 1985, entitled "Olefin Epoxidation in a Polar Medium" (D # 80,313-Cl). After distillation of the propylene oxide, the crude tert-butyl alcohol (TBA) fraction was obtained by distillation contained > 90% TBA. This crude TBA fraction contained 1.28% tert-butyl hydroperoxide (TBHP), 0.628% di-tert-butyl peroxide (DTBP) along with small amounts of acetone, methanol and other impurities. < 0.1% isobutane was present.

### Example 1

Batch Screening Experiments (Only Contaminant, Tertiary Butyl Hydroperoxide)

A small stainless steel rocker autoclave fitted with a glass liner was charged with 80 ml of a simulated motor fuel grade tertiary butyl alcohol feedstock containing the impurities listed under 5936-37 in the amount indicated. The simulated feedstock was formulated to contain impurities and impurity levels representative of a motor fuel grade tertiary butyl alcohol recovered from a reaction mixture in the manner described above in Example A. Also, the simulated motor fuel grade tertiary butyl alcohol provided a standard for use in comparing the effectiveness of the catalysts that were tested.

Catalyst was added and the autoclave was pressured with hydrogen. The reaction mixture was then heated to the desired temperature for the desired amount of time. The reaction mixture was cooled to ambient temperature, vented and the catalyst filtered from the clear solution.

The catalysts tested and the results obtained are set forth in the following tables.

6

## TABLE I

### CATALYTIC REDUCTION OF TERTIARY BUTYL HYDROPEROXIDE USING SUPPORTED NICKEL CATALYSTS

| Run No. | Notebook No. | Catalyst | GM | Press (psig/kg/cm² Temp °C | Time Hr | TBHP[a] | TBA | Products by GC, % Acet. | IPA | IB | Unk |
|---|---|---|---|---|---|---|---|---|---|---|---|
| 1 | 5936-37 | Simulated Feed | | | | 2.13 | 99.621 | .028 | .199 | .004 | .071 |
| 2 | 5898-89 | (b) | 2.0 | (1000) 70 125 | 2.0 | .004 | 99.551 | .295 | .295 | .015 | .043 |
| 3 | 5916-15 | (b) | 2.0 | (650) 45 125 | 2.0 | .006 | 99.460 | .102 | .280 | .033 | .046 |
| 4 | 5916-16 | (b) | 2.0 | (575) 40 80 | 2.0 | .030 | 99.401 | .194 | .213 | .011 | .063 |
| 5 | 5916-17 | (b) | 1.0 | (600) 42 125 | 2.0 | .020 | 99.612 | .027 | .247 | .014 | .046 |
| 6 | 5916-18 | (b) | .54 | (725) 51 125 | 2.0 | .020 | 99.557 | .110 | .220 | 0 | .063 |
| 7 | 5916-29 | (c) | 2.0 | (650) 45 125 | 2.0 | 2.04 | 99.519 | .140 | .152 | .016 | .071 |
| 8 | 6916-77 | (d) | 2.0 | (550) 38 125 | 2.0 | 0 | 97.565 | .009 | .255 | 1.856 | .093 |
| 9 | 5916-78 | (e) | 2.0 | (650) 45 125 | 2.0 | .74 | 99.093 | .336 | .140 | .117 | .028 |
| 10 | 5936-11 | (f) | 2.0 | (585) 41 125 | 2.0 | 0 | 99.362 | .019 | .415 | .065 | .029 |
| 11 | 5936-12 | (g) | 2.0 | (650) 45 125 | 2.0 | .020 | 99.047 | .010 | .311 | .315 | .147 |
| 12 | 5936-77 | (f) | 2.0 | (650) 45 125 | 2.0 | .050 | 99.286 | .402 | .171 | .004 | .013 |
| 13 | 5936-78 | (f) | 2.0 | (620) 43 80 | 2.0 | .620 | 99.455 | .198 | .151 | .002 | .036 |

TABLE I (Cont.)

| Run No. | Notebook No. | Catalyst | GM | Press (psig) | Temp °C | Time Hr | TBHP[a] | Products by GC, % | | | | |
|---|---|---|---|---|---|---|---|---|---|---|---|---|
| | | | | | | | | TBA | Acet. | IPA | IB | Unk |
| 14 | 5936-79 | (h) | 2.0 | (675) 47 | 125 | 2.0 | .120 | 99.282 | .444 | .141 | .003 | .006 |
| 15 | 5936-80 | (h) | 2.0 | (600) 42 | 80 | 2.0 | 1.18 | 99.459 | .190 | .154 | .007 | .040 |
| 16 | 5973-93 | E-480P (Ni R+S)(i) | 2.0 | (400) 28 | 80 | 2.0 | 0 | 99.519 | .008 | .294 | .013 | .075 |

(a) Weight % TBHP determined by iodometric titration
(b) Ni, Cu, Cr catalyst similar to the one described in U. S. Patent 3,654,370
(c) 8% Ni on Spinel
(d) Zr promoted Ni on Kieselguhr
(e) NiO on $SiO_2$
(f) Ni on Kieselguhr
(g) Ni on $SiO_2/Al_2O_3$
(h) Nickel on refractory
(i) Nickel catalyst partially reduced

TABLE II

CATALYTIC REDUCTION OF TERTIARY BUTYL HYDROPEROXIDE
USING SUPPORTED COBALT CATALYSTS

| Run No. | Notebook No. | Catalyst | GM | Press (psig) | Temp C | Time Hr | TBHP[a] | Products by GC, % | | | | |
|---|---|---|---|---|---|---|---|---|---|---|---|---|
| | | | | | | | | TBA | Acet. | IPA | IB | Unk |
| 1 | 5936-37 | Simulated Feed | | | | | 2.13 | 99.621 | .028 | .199 | .004 | .071 |
| 2 | 5898-90 | (b) | 2.0 | (1125)77 | 125 | 2.0 | .390 | 99.449 | .009 | .420 | 0 | .036 |
| 3 | 5916-30 | (c) | 2.0 | (750)52 | 125 | 2.0 | .020 | 99.149 | .490 | .155 | .006 | .063 |
| 4 | 5916-94 | (d) | 2.0 | (650)45 | 125 | 2.0 | 1.04 | 99.169 | .397 | .120 | .020 | .069 |
| 5 | 5936-47 | (c) | 2.0 | (325)23 | 125 | 2.0 | .015 | 99.201 | .011 | .445 | .036 | .062 |
| 6 | 5936-48 | (c) | 2.0 | (600)42 | 80 | 2.0 | .050 | 98.771 | .382 | .581 | .009 | .067 |
| 7 | 5936-49 | (c) | 1.0 | (700)41 | 125 | 2.0 | .030 | 99.141 | .401 | .187 | .027 | .064 |
| 8 | 5936-50 | (c) | .50 | (700)49 | 125 | 2.0 | .020 | 99.041 | .552 | .130 | .009 | .065 |
| 9 | 5936-75 | (e) | 2.0 | (650)45 | 125 | 2.0 | .020 | 98.734 | .327 | .646 | .021 | .052 |
| 10 | 5936-76 | (e) | 2.0 | (575)40 | 80 | 2.0 | .060 | 99.327 | .261 | .161 | .003 | .065 |

(a) Weight % TBHP determined by iodometric titration
(b) Co, Cu, Cr catalyst that has approximately 75 g. atom % cobalt, 23 g. atom % copper and 2 g. atom % chromium
(c) Co on Kieselguhr
(d) Co on Silica
(e) Zr promoted Co on Kieselguhr

TABLE III

## CATALYTIC REDUCTION OF TERTIARY BUTYL HYDROPEROXIDE USING SUPPORTED PALLADIUM CATALYSTS

| Run No. | Notebook No. | Catalyst | GM | Press psig / Temp C | Time Hr | TBHP[a] | TBA | Acet. | IPA | IB | Unk |
|---|---|---|---|---|---|---|---|---|---|---|---|
| 1 | 5936-37 | Simulated Feed | | | | 2.13 | 99.621 | .028 | .199 | .004 | .071 |
| 2 | 5936-33 | 1 % Pd on C | 2.0 | (525) 37  80 | 2.0 | .010 | 99.623 | .066 | .190 | 0 | .066 |
| 3 | 5936-34 | 1 % Pd on C | 1.0 | (550) 38  80 | 2.0 | .020 | 99.501 | .192 | .191 | .001 | .066 |
| 4 | 5936-35 | 1 % Pd on C | .50 | (600) 42  80 | 2.0 | .006 | 99.654 | .032 | .190 | 0 | .067 |
| 5 | 5936-36 | 1 % Pd on C | 2.0 | (300) 21  125 | 2.0 | .009 | 99.690 | .017 | .183 | .014 | .054 |
| 6 | 5916-76 | 1 % Pd on C | 2.0 | (700) 41  125 | 2.0 | 0 | 99.670 | .025 | .183 | .016 | .056 |
| 7 | 5959-37 | 1 % Pd on Graphite | 2.0 | (335) 23  80 | 2.0 | .010 | 99.536 | .048 | .188 | .098 | .067 |
| 8 | 5959-38 | 5 % Pd on BASO4 | 2.0 | (350) 24  80 | 2.0 | .020 | 99.639 | .080 | .156 | .016 | .066 |
| 9 | 5959-33 | 5 % Pd on CaCO3 | 2.0 | (350) 24  80 | 2.0 | .010 | 99.674 | .035 | .180 | .009 | .062 |
| 10 | 5916-96 | 5 % Pd on Al2O3 | 2.0 | (695) 41  125 | 2.0 | .020 | 99.605 | .016 | .192 | 0 | .057 |
| 11 | 5959-36 | 5 % Pd on BaCO3 | 2.0 | (355) 25  80 | 2.0 | .040 | 99.650 | .031 | .185 | .001 | .067 |
| 12 | 5973-95 | E-251 (5% Pd on C) | 2.0 | (325) 23  80 | 2.0 | .006 | 99.713 | .024 | .165 | .005 | .049 |
| 13 | 5959-35 | Pd on Al2O3, | 2.0 | (425) 30  80 | 2.0 | .007 | 99.725 | .043 | .172 | .002 | .011 |
| 14 | 5959-34 | Pd on CaCO3 (Pb Poison) | 2.0 | (350) 24  80 | 2.0 | .008 | 99.549 | .065 | .177 | 0 | .067 |
| 15 | 5916-97 | 5 % Pd on Al2O3 | 2.0 | (650) 45  125 | 2.0 | 0 | 99.594 | .040 | .175 | – | – |
| 16 | 6064-19 | 5 % Pt on C | 2.0 | (570) 40  80 | 2.0 | .001 | 98.903 | .007 | .478 | .075 | .438 |
| 17 | 6064-20 | 5 % Pt on C | 2.0 | (600) 42  120 | 2.0 | .001 | 98.590 | .010 | .944 | .312 | .059 |

(a) Weight % TBHP determined by iodometric titration

TABLE IV

CATALYTIC REDUCTION OF TERTIARY BUTYL HYDROPEROXIDE
USING COPPER CHROMITE CATALYSTS

| Run No. | Notebook No. | Catalyst | GM | Press (psig)Kj/c. | Temp °C | Time Hr | TBHP[a] | TBA | Acet. | IPA | IB | Unk |
|---------|--------------|----------|-----|-------------------|---------|---------|---------|---------|-------|------|------|------|
| 1 | 5936-37 | Simulated Feed | | | | | 2.13 | 99.621 | .028 | .199 | .004 | .071 |
| 2 | 5959-1 | (b) | 2.0 | (635)44 | 125 | 2.0 | .020 | 99.320 | .008 | .512 | .010 | .054 |
| 3 | 5959-2 | (b) | 2.0 | (600)42 | 80 | 2.0 | .25 | 99.366 | .335 | .125 | .001 | .060 |
| 4 | 5959-3 | (c) | 2.0 | (675)47 | 125 | 2.0 | .030 | 99.259 | .078 | .517 | .005 | .058 |
| 5 | 5959-4 | (c) | 2.0 | (600)42 | 80 | 2.0 | 1.33 | 99.531 | .177 | .150 | .003 | .068 |
| 6 | 5973-96 | (d) | 2.0 | (375)26 | 80 | 2.0 | .270 | 99.366 | .345 | .116 | .001 | .059 |
| 7 | 5973-97 | (e) | 2.0 | (375)26 | 80 | 2.0 | .270 | 99.312 | .364 | .125 | .004 | .064 |
| 8 | 5973-98 | (f) | 2.0 | (385)27 | 80 | 2.0 | .170 | 99.185 | .481 | .112 | .003 | .063 |

(a)  Weight % TBHP determined by iodometric titration
(b)  Nonpromoted copper chromite
(c)  Copper chromite on silica
(d)  Copper chromite: 39% CuO, 40% $Cr_2O_3$
(e)  Copper chromite: 47% CuO, 47% $Cr_2O_3$
(f)  Promoted copper chromite

## TABLE V

### CATALYTIC REDUCTION OF TERTIARY BUTYL HYDROPEROXIDE USING SUPPORTED MISCELLANEOUS CATALYSTS

| Run No. | Notebook No. | Catalyst | GM | Press (psig)$k/m^3$ | Temp °C | Time Hr | TBHP[a] | Products by GC, % | | | | |
|---|---|---|---|---|---|---|---|---|---|---|---|---|
| | | | | | | | | TBA | Acet. | IPA | IB | Unk |
| 1 | 5936–37 | Simulated Feed | | | | | 2.13 | 99.621 | .028 | .199 | .004 | .071 |
| 2 | 5936–63 | (b) | 2.0 | (700) 49 | 125 | 2.0 | .130 | 98.800 | .864 | .097 | .006 | .066 |
| 3 | 5936–64 | (b) | 2.0 | (595) 42 | 80 | 2.0 | 2.43 | 99.099 | .091 | .673 | .004 | .069 |
| 4 | 5936–81 | (c) | 2.0 | (700) 49 | 125 | 2.0 | .090 | 98.890 | .732 | .123 | .016 | .065 |
| 5 | 5936–82 | (c) | 2.0 | (600) 42 | 80 | 2.0 | 1.35 | 99.526 | .212 | .126 | .010 | .067 |
| 6 | 5916–93 | (d) | 2.0 | (700) 49 | 125 | 2.0 | 0 | 98.718 | .011 | .416 | .023 | .014 |
| 7 | 5936–61 | (e) | 2.0 | (650) 45 | 125 | 2.0 | .230 | 99.215 | .415 | .122 | .011 | .026 |
| 8 | 5936–62 | (e) | 2.0 | (585) 41 | 80 | 2.0 | 1.18 | 99.048 | .157 | .635 | .003 | .045 |

(a) Weight % TBHP determined by iodometric titration
(b) Cu, Zn, Cr catalyst
(c) Cu, Cd, Cr catalyst
(d) Ni, Cu, Cr, Fe on Kieselguhr
(e) 0.55% Ru on Alumina

As will be seen from Table I through Table V, erratic results were obtained with the catalysts screened insofar as the reduction of tertiary butyl hydroperoxide to tertiary butyl alcohol is concerned.

0 215 588

12

For example, and with reference to Table I (supported nickel catalysts), either there was a poor to moderate reduction of the TBHP or an unsatisfactory increase in by-product formation. In runs 5896-89 through 5916-18, acetone and IPA levels increased. In run 5916-29, there was only minimal reduction of TBHP, while in run 6916-77, the TBHP was completely reduced, but significant quantities of IPA and IB were formed.

Also, in runs 5916-78, 5936-77, 5936-78, 5936-79 and 5973-94 there was inadequate reduction of TBHP whereas in the remainder of the runs there was an undesirable increase in the level of acetone and/or IPA and/or IB in the product.

Turning next to Table II summarizing the experiments conducted for the purpose of screening cobalt catalysts, it is seen that the same erratic pattern of results was obtained in that in the runs where a reasonable reduction of the TBHP was obtained, there was an undesirable increase in the acetone level (runs 5916-30, 5936-48, 5936-49, 5936-50, 5936-75 and 5936-70) or in the level of isopropyl alcohol. The same observation holds true for Tables IV and V wherein copper chromite and other copper catalysts and ruthenium or alumina were screened.

With respect to Table IV, compare 5959-1 and 5959-2. At the higher temperature 125°C, the % TBHP is reduced to .02 wt.% and there is not a large quantity of acetone indicated. There is however, a large quantity of IPA formed. This IPA results from the reduction of acetone. There is no other reasonable way for the IPA to be formed. Thus, there is little IPA indicated in 5959-2, a larger quantity of acetone and the TBHP is not reduced as low as 5959-1. Essentially the same results are obtained with 5959-3 and 5959-4 with a different catalyst. The other catalysts in this table do not reduce TBHP to a low level and a large quantity of acetone is formed. It is desirable to reduce TBHP to TBA, not decompose it and then reduce the acetone.

The only group of catalysts that was effective for the selective reduction of TBHP without excessive increases in the level of acetone, isopropyl alcohol and isobutane were the palladium catalysts of Table III.

Example 2

Catalytic Reduction of TBHP and DTBP Using Various Catalysts

A series of tests were conducted in a continuous 100 cc stainless steel tubular reactor connected to a tubular inlet bottom line and a tubular outlet upper line. Temperature control was maintained by wrapping the reactor with 1000 watt strip heaters which, together with monitoring thermocouples attached to the skin of the reactor body, were interconnected with a thermoelectric controller. Hydrogen and liquid feed were pumped into the bottom of the reactor and pressure was regulated with a uni-flow valve. All catalysts were prereduced in the reactor prior to test runs. Each test run was conducted for an initial period of about 2 hours to obtain "lined out" reaction conditions and the run was continued for an additional 0.5 to 4.0 hour sampling period.

The composition of a simulated feedstock containing both TBHP and DTBP, as well as isobutane, acetone and isopropyl alcohol is given in Table VI together with a listing of the reaction conditions employed, the catalysts tested and the results obtained. Note that in contrast to Tables I through V, % TBHP results are reported on the basis of PPM.

TABLE VI

CATALYTIC REDUCTION OF A SIMULATED TERTIARY
BUTYL ALCOHOL FEEDSTOCK CONTAINING TBHP AND
DTBP USING VARIOUS CATALYSTS.

| Notebook No. | Liquid Feed (cc/hr) | $H_2$ Feed (cc/hr) | Temp. (°C) | Reactor Pressure; $Kg/km^2$ (psig) | Catalyst |
|---|---|---|---|---|---|
| 6021-40-1 | 48-50 | 388 | 80 | 2-8 (40) | 40% Ni on $Al_2O_3$-1/16" Spheres (Calsicat) |
| -2 | 48-50 | 388 | 150 | 2-9 (40) | |
| -3 | 48-50 | 388 | 80 | 11-2 (160) | |
| -4 | 48-50 | 388 | 150 | 11-1 (160) | |
| 6021-46-1 | 49-50 | 388 | 80 | 2-3 (40) | 0.5% Pd on $Al_2O_3$ (Calsicat) |
| -2 | 49-50 | 388 | 80 | 11-2 (160) | |
| -3 | 49 | 388 | 150 | " (160) | |
| 6021-06-1 | 49-50 | 388 | 80 | " (160) | 0.2% Pd on Carbon (United) |
| -2 | 46-50 | 388 | 100 | " (160) | |
| -3 | 49-50 | 388 | 120 | " (160) | |
| 6021-65-1 | 100 | 200 | 40 | " (160) | 0.41% Pd on $Al_2O_3$ (United) |
| -2 | 100 | 200 | 80 | " (160) | |
| 6021-70-1 | 100 | 200 | 40 | " (160) | 40% Ni on $Al_2O_3$ (Calsicat) |
| -2 | 100 | 200 | 80 | " (160) | |
| 5787-65-1 | - | - | - | - - | Simulated feed, starting material for reductions 6021-40, -46, -06, -65 and -70 |

13

TABLE VI (Cont.)

| Notebook No. | TBHP by Titr. (PPM) | H$_2$O by IR (wt.%) | Products by GC | | | |
|---|---|---|---|---|---|---|
| | | | IB (wt.%) | DTBP (wt.%) | Acetone (wt.%) | IPA (wt.%) |
| 6021-40-1 | 4.26 | 2.1 | 0.003 | 0 | 0.005 | 0.235 |
| -2 | 5.34 | 2.1 | 0.411 | 0.007 | 0.113 | 0.039 |
| -3 | 3.33 | - | 0.006 | 0 | 0 | 0.230 |
| -4 | 5.26 | - | 0.214 | 0.019 | 0.152 | 0.027 |
| 6021-46-1 | 3.70 | 1.8 | 0.003 | 0.041 | 0.038 | 0.208 |
| -2 | 5.11 | 1.8 | 0.003 | 0.041 | 0.048 | 0.202 |
| -3 | 3.35 | 1.9 | 0.075 | 0.040 | 0.056 | 0.206 |
| 6021-06-1 | 3.83 | 1.9 | 0.031 | 0.052 | 0.043 | 0.205 |
| -2 | 3.18 | 1.9 | 0.071 | 0.014 | 0.045 | 0.215 |
| -3 | 2.40 | 2.2 | 0.282 | 0.010 | 0.058 | 0.208 |
| 6021-65-1 | 6.43 | 1.8 | 0.005 | 0.107 | 0.041 | 0.207 |
| -2 | 4.35 | 1.9 | 0.002 | 0.054 | 0.035 | 0.209 |
| 6021-70-1 | 3.21 | 1.6 | 0.004 | 0.091 | 0.022 | 0.208 |
| -2 | 4.16 | 1.7 | 0.004 | 0.004 | 0.025 | 0.209 |
| 5787-65-1 | 887 | 2.0 | 0.008 | 0.135 | 0.032 | 0.219 |

Note from Table VI that satisfactory reduction of the TBHP content to less than 10 ppm was obtained in all instances. A reduction in the level of DTBP to less than .02 wt.% was uniformly attained only with the nickel on alumina catalyst of runs 6021-40-1 through 6021-40-4. Even for these runs, a reaction temperature of 150°C was excessive, as shown by the increase in isobutane and acetone concentration. Runs 6021-70-1 and 6021-70-2 show that for this particular catalyst a reaction temperature of 40°C was too low in that the DTBP concentration was more than 0.02 wt.% in run 6021-70-1 but within the acceptable limit when the temperature was raised to 80°C in run 6021-70-2.

In contrast, palladium supported on alumina did not reduce the DTBP concentration to less than 0.02 wt.%. Although palladium on charcoal reduced the DTBP level to less than 0.02 wt.% in runs 6021-06-2 and 6021-06-3 at reaction temperatures of 100°C and 120°C, respectively, the results, overall, were not satisfactory. principally because of the increase in isobutane content, but also also because of the increase in acetone content. When the temperature was lowered (run 6021-06-1) the reduction of DTBP was inadequate.

14

## TABLE VII

### CATALYTIC REDUCTION OF A SIMULATED TERTIARY BUTYL ALCOHOL FEEDSTOCK CONTAINING TBHP AND DTBP USING NICKEL ON SILICA ALUMINA CATALYSTS

| Notebook No. | Liquid Feed (cc/hr) | $H_2$ Feed (cc/hr) | Temp. (°C.) | Reactor Pressure $Kg/cm^2$ (psig) | Catalyst |
|---|---|---|---|---|---|
| 6021-57-1 | 45-49 | 388 | 80 | 2.8 (40) | 56% Ni on $SiO_2/Al_2O_3$ (Less than 50% $Al_2O_3$) |
| -2 | 49-51 | 388 | 80 | 11.2 (160) | |
| -3 | 47-51 | 388 | 150 | 11.2 (160) | |
| 6021-32-1 | 49-50 | 388 | 80 | 2.8 (40) | 50% Ni on $SiO_2/Al_2O_3$ (Over 50% $Al_2O_3$) |
| -2 | 48-50 | 388 | 150 | 2.8 (40) | *Note this catalyst |
| -3 | 48-50 | 388 | 80 | 11.2 (160) | has a higher ratio of $Al_2O_3$ to $SiO_2$ than |
| -4 | 48-52 | 388 | 150 | " (160) | first catalyst above |
| 6021-60-1 | 100 | 200 | 40 | " (160) | 56% Ni on $SiO_2/Al_2O_3$ (Less than 50% $Al_2O_3$) |
| -2 | 100 | 200 | 80 | " (160) | * |
| 6021-74-1 | 100 | 200 | 40 | " (160) | 50% Ni on $SiO_2/Al_2O_3$ (Less than 50% $Al_2O_3$) |
| -2 | 100 | 200 | 80 | " (160) | |
| 6021-36-1 | 47-50 | 388 | 80 | 2.8 (40) | 58% Ni on Kieselguhr |
| -2 | 48-52 | 388 | 150 | 2.8 (40) | |
| -3 | 48-50 | 388 | 80 | 11.2 (160) | |
| -4 | 55-49 | 388 | 150 | 11.2 (160) | |
| 5787-65-1 | - | - | - | - | Simulated feed, starting material for reductions 6021-57, -32, -60, -74 and -36 |

# 0 215 588

## TABLE VII (Cont.)

| Notebook No. | TBHP by Titr. (PPM) | H₂O by IR (wt.%) | IB (wt.%) | DTBP (wt.%) | Acetone (wt.%) | IPA (wt.%) |
|---|---|---|---|---|---|---|
| 6021-57-1 | 6.46 | 2.0 | 0.404 | 0.040 | 0.010 | 0.227 |
| -2 | 4.76 | 2.0 | 0.541 | 0.045 | 0.112 | 0.224 |
| -3 | 6.37 | 2.2 | 4.497 | 0.061 | 0.342 | 0.005 |
| 6021-32-1 | 3.10 | 1.8 | 0.003 | 0 | 0 | 0.234 |
| -2 | 3.90 | 2.1 | 0.164 | 0.016 | 0.159 | 0.076 |
| -3 | 4.61 | 1.9 | 0.002 | 0.001 | 0 | 0.237 |
| -4 | 3.70 | 2.3 | 0.155 | 0.025 | 0.159 | 0.041 |
| 6021-60-1 | 3.46 | 1.9 | 0.045 | 0.131 | 0.051 | 0.210 |
| -2 | 3.92 | 1.9 | 0.064 | 0.092 | 0.054 | 0.199 |
| 6021-74-1 | 4.06 | 1.8 | 0.004 | 0.060 | 0.031 | 0.208 |
| -2 | 3.86 | 1.8 | 0.005 | 0.003 | 0.035 | 0.203 |
| 6021-36-1 | 3.34 | 1.8 | 0.007 | 0.023 | 0.009 | 0.240 |
| -2 | 4.06 | 5.0 | 1.687 | 0.015 | 0.330 | 0 |
| -3 | 3.54 | 1.9 | 0.004 | 0.089 | 0.021 | 0.247 |
| -4 | 3.81 | 2.6 | 2.504 | 0.085 | 0.317 | 0.007 |
| 5787-65-1 | 887 | 2.0 | 0.008 | 0.135 | 0.032 | 0.219 |

Example 8

Preparation of Motor Fuel Grade Tertiary Butyl Alcohol from Tertiary Butyl Hydroperoxide and Propylene
In a representative run, a 300 ml autoclave was charged with 127.5 grams of a feed composed of 72.14% t-butyl hydroperoxide, 0.38% water and 27.48% t-butyl alcohol and minor amounts of other components. 0.49 Grams of molybdenum-ethylene glycol complex containing 13.2% of molybdenum were added and then 48 grams of propylene were charged. This mixture of reactants and catalyst were reacted for 2 hours at 105°C and autogenous pressure 49kg/cm² (600 psig)max. A sample of the reaction mixture was pressured from the autoclave, analyzed and found to contain 7.4% propylene, 0.4% water, 0.1% methanol, 26.3% propylene oxide, 0.2% acetone, 0.1% isopropyl alcohol, 64.9% t-butyl alcohol/t-butyl hydroperoxide, 0.5% propylene glycol and 0.2% heavies. Ditertiary butyl hydroperoxide was not detected.
Thereafter, the reaction mixture was stripped at 35°C and atmospheric pressure for about 0.5 hours. The stripped reaction product was analyzed and found to contain 1.2% propylene, 18.5% propylene oxide, 65.9% t-butyl alcohol, 11.6% t-butyl hydroperoxide, 0.6% methanol, 0.1% acetone, 0.1% isopropyl alcohol and 0.1% heavies.
Tertiary butyl alcohol recovered from the stripped reaction mixture by distillation will still contain contaminating quantities of t-butyl hydroperoxide, methanol, acetone and isopropyl alcohol.

16

Example 3

A small stainless steel rocker autoclave fitted with a glass liner was charged with 80 ml of a simulated motor fuel grade tertiary butyl alcohol feedstock to which known quantities of impurities were added (Table I). The simulated feedstock was formulated to contain impurities and impurity levels representative of a motor fuel grade tertiary butyl alcohol recovered by distillation from a stripped reaction mixture of the type described in Example 3 above. Also, the simulated motor fuel grade tertiary butyl alcohol provided a standard for use in comparing the effectiveness of the catalysts that were tested.

Catalysts was added and the autoclave was pressured with hydrogen. The reaction mixture was then heated to the desired temperature for the desired amount of time. The reaction mixture was cooled to ambient temperature, vented and the catalyst filtered from the clear solution.

The catalysts tested and the results obtained are set forth in the following table.

17

## TABLE VIII

### CATALYTIC REDUCTION OF TERTIARY BUTYL HYDROPEROXIDE USING SUPPORTED PALLADIUM CATALYSTS

| Run No. | Notebook No. | Catalyst | GM | Press (psig)/°C | Temp °C | Time Hr | TBHP[a] | TBA | Acet. | IPA | IB | Unk |
|---|---|---|---|---|---|---|---|---|---|---|---|---|
| 1 | 5936-37 | Simulated Feed | | | | | 2.13 | 99.621 | .028 | .199 | .004 | .071 |
| 2 | 5936-33 | 1 % Pd on C | 2.0 | (525) 37 | 80 | 2.0 | .010 | 99.623 | .066 | .190 | 0 | .066 |
| 3 | 5936-34 | 1 % Pd on C | 1.0 | (550) 33 | 80 | 2.0 | .020 | 99.501 | .192 | .191 | .001 | .066 |
| 4 | 5936-35 | 1 % Pd on C | .50 | (600) 42 | 80 | 2.0 | .006 | 99.654 | .032 | .190 | 0 | .067 |
| 5 | 5936-36 | 1 % Pd on C | 2.0 | (300) 21 | 125 | 2.0 | .009 | 99.690 | .017 | .183 | .014 | .054 |
| 6 | 5916-76 | 1 % Pd on C | 2.0 | (700) 47 | 125 | 2.0 | 0 | 99.670 | .025 | .183 | .016 | .056 |
| 7 | 5959-37 | 1 % Pd on Graphite | 2.0 | (335) 23 | 80 | 2.0 | .010 | 99.536 | .048 | .188 | .098 | .067 |
| 8 | 5959-38 | 5 % Pd on BaSO4 | 2.0 | (350) 24 | 80 | 2.0 | .020 | 99.639 | .080 | .156 | .016 | .066 |
| 9 | 5959-33 | 5 % Pd on CaCO3 | 2.0 | (350) 24 | 80 | 2.0 | .010 | 99.674 | .035 | .180 | .009 | .062 |
| 10 | 5916-96 | 5 % Pd on Al2O3 | 2.0 | (695) 47 | 125 | 2.0 | .020 | 99.605 | .016 | .192 | 0 | .057 |
| 11 | 5959-36 | 5 % Pd on BaCO3 | 2.0 | (355) 25 | 80 | 2.0 | .040 | 99.650 | .031 | .185 | .001 | .067 |
| 12 | 5973-95 | E-251 (5% Pd on C) | 2.0 | (325) 23 | 80 | 2.0 | .006 | 99.713 | .024 | .165 | .005 | .049 |
| 13 | 5959-35 | Pd on Al2O3 | 2.0 | (425) 30 | 80 | 2.0 | .007 | 99.725 | .043 | .172 | .002 | .011 |
| 14 | 5959-34 | Pd on CaCO3 (Pb Poison) | 2.0 | (350) 24 | 80 | 2.0 | .008 | 99.549 | .065 | .177 | 0 | .067 |
| 15 | 5916-97 | 5 % Pd on Al2O3 | 2.0 | (650) 45 | 125 | 2.0 | ~0 | 99.594 | .040 | .175 | - | - |
| 16 | 6064-19 | 5 % Pt on C | 2.0 | (570) 40 | 80 | 2.0 | .001 | 98.903 | .007 | .478 | .075 | .438 |
| 17 | 6064-20 | 5 % Pt on C | 2.0 | (600) 41 | 120 | 2.0 | .001 | 98.590 | .010 | .944 | .312 | .059 |

(a) Weight % TBHP determined by iodometric titration

As will be seen from Table VIII, in all instances there was a significant reduction of the tertiary butyl hydroperoxide content of the tertiary butyl alcohol feedstock such that substantially all of the tertiary butyl

18

0 215 588

hydroperoxide was destroyed. Note also, by comparing control Run No. 1 under the column acetone with the other runs that the acetone content was either decreased or only insignificantly increased in all instances except for Run No. 5936-35.

Note also that the isopropyl alcohol content was either decreased or only insignificantly increased by the process of the present invention, as was also the case with isobutane.

Example 4

The results obtained through the process of the present invention are not obtainable with other catalyst compositions.

For example, when the experiment was repeated using 2 grams of a catalyst consisting of cobalt supported on silica at a pressure of 45 kg/cm$^2$ (650 psig) and a temperature of 125°C for two hours, the resultant product still contained 1.04 wt.% of tertiary hydroperoxide and also 0.397 wt.% of acetone and 0.120 wt.% of isopropyl alcohol.

When a catalyst consisting essentially of copper oxide and chromium oxide supported on silica was utilized at a pressure of 51 kg/cm$^2$ (725 psig), a temperature of 125°C with a reaction time of 2 hours, the tertiary butyl hydroperoxide was effectively decomposed providing a reaction product containing only 0.035 wt % of tertiary butyl hydroperoxide. However, the acetone content was 0.429 wt.% and the isopropyl alcohol content was 0.239 wt %.

As another example, a commercial catalyst comprising copper, zinc and chromium was used. When the reaction was conducted for two hours at a pressure of 49 kg/cm$^2$ (700 psig) and a reaction temperature of 125°C, the reaction product contained 0.130 wt % of tertiary butyl hydroperoxide, 0.864 wt % of acetone and 0.097 wt % of isopropyl alcohol. With the same catalyst at a reaction pressure of 42 kg/cm$^2$ (595 psig) and a reaction temperature of 80°C for a reaction time of 2 hours, a product was obtained containing 2.43 wt % of tertiary butyl hydroperoxide, 0.091 wt % of acetone and 0.673 wt % of isopropyl alcohol.

## Claims

1. A method for enhancing the motor fuel quality of a tertiary butyl alcohol feedstock obtained by the reaction of propylene with tertiary butyl hydroperoxide to form propylene oxide and tertiary butyl alcohol contaminated with residual quantities of tertiary butyl hydroperoxide, and ditertiary butyl peroxide where present; Characterised by contacting said feedstock with a supported catalyst in the presence of hydrogen under mild catalytic conditions including a temperature of about 50° to 150°C, a maximum pressure of 70 kg/cm$^2$ (1000 psig) correlated to substantially selectively reduce said hydroperoxides to tertiary butyl alcohol and recovering substantially hydroperoxide-free tertiary butyl alcohol from the reaction products;
wherein the catalyst is selected from; a nickel catalyst composition containing 5 to 75 wt % of the active catalytic metal component, and wherein said support comprises 50 to 100 wt % of alumina, and correspondingly 50 to 0 wt % of silica, and the minimum pressure is about 10.5 kg/cm$^2$ (150 psig);
or
a platinum and/or palladium catalyst composition containing 0.01 wt % to 5 wt % of active catalytic metal component, wherein the minimum pressure is 21 kg/cm$^2$ (300 psig).

2. A method as in claim 1 wherein added hydrogen is present in an amount of 1.1 to 5.0 mol %, based on said hydroperoxide, and diperoxide where present.

3. A method as in claims 1 or 2 wherein the temperature is within the range of about 70° to about 130° C and the pressure has a value of up to about 700 psig.

4. A method as in any preceding claim wherein the catalyst support comprises alumina.

5. A method as in claims 1 to 3 wherein the catalyst support comprises both silica and alumina and containing not less than about 50 wt % of alumina.

6. A method according to claim 1 wherein the catalytic support has 0.5 wt % to 5 wt % palladium deposited thereon and the support is selected from charcoal, calcium carbonate, barium carbonate, barium sulphate, and graphite.

7. A method according to claim 1 wherein the catalytic support has 0.5 wt % to 5 wt % of platinum deposited thereon and the support is carbon.